(19) Europäisches Patentamt
European Patent Office
Office européen des brevets

(11) Veröffentlichungsnummer: **0 045 371**
**B1**

# (12) EUROPÄISCHE PATENTSCHRIFT

(45) Veröffentlichungstag der Patentschrift: **22.02.84**

(51) Int. Cl.³: **C 07 D 249/12**

(21) Anmeldenummer: **81104769.5**

(22) Anmeldetag: **22.06.81**

## (54) Verfahren zur Herstellung von in 4-Stellung monosubstituierten 1,2,4-Triazolidin-3,5-dionen.

(30) Priorität: **21.07.80 DE 3027612**

(43) Veröffentlichungstag der Anmeldung: **10.02.82 Patentblatt 82/6**

(45) Bekanntmachung des Hinweises auf die Patenterteilung: **22.02.84 Patentblatt 84/8**

(84) Benannte Vertragsstaaten: **CH DE FR GB IT LI**

(56) Entgegenhaltungen:
**EP - A - 0 030 627**
**FR - A - 1 447 532**
**FR - A - 1 470 310**
**FR - A - 2 061 024**

**CHEMICAL ABSTRACTS, Band 67, Nr. 25, 18. Dezember 1967, Seite 11009, Zusammenfassung 116858y COLUMBUS OHIO (US) & Chem. Zvesti 21 (6), 427-42 (1967) M. FURDIK et al "Synthesis of azodicarboxylic acid imide, its N-substituted derivatives and their use as dienophiles in the Diels-Alder reaction"**
**CANADIAN JOURNAL OF CHEMISTRY, Band 50, Nr. 11, 1972 J.A. LENOIR et al "Hydrazine derivatives. I. Transcarbamylation reaction", Seiten 2661-2666**

**Die Akte enthält technische Angaben, die nach dem**

(73) Patentinhaber: **BAYER AG, Zentralbereich Patente, Marken und Lizenzen, D-5090 Leverkusen 1, Bayerwerk (DE)**

(72) Erfinder: **Giesecke, Henning, Dr., Kalk-Muelheimer Strasse 400, D-5000 Koeln 80 (DE)**
Erfinder: **Merten, Rudolf, Dr., Berta-von-Suttner-Strasse 55, D-5090 Leverkusen (DE)**
Erfinder: **Rottmaier, Ludwig, Bergstrasse 85, D-5068 Odenthal (DE)**

(56) Entgegenhaltungen: (Fortsetzung)
**Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

Verfahren zur Herstellung von in 4-Stellung monosubstituierten 1,2,4-Triazolidin-3,5-dionen

Die Erfindung betrifft Verfahren zur Herstellung von 1,2,4-Triazolidin-3,5-dionen, die in 4-Stellung durch gegebenenfalls substituierte Kohlenwasserstoffreste, welche durch Heteroatome oder Atomgruppen unterbrochen sein können, substituiert sind, wie unten definiert ist.

Die Herstellung von 4-Phenyl-1,2,4-triazolidin-3,5-dion aus Hydrazodicarbonamid und Anilinhydrochlorid ist bekannt. Sie wird, wie in Liebigs Ann. 283, 41 (1894) beschrieben, durch Erhitzen eines Gemisches aus Hydrazodicarbonamid und Anilinhydrochlorid in der Schmelze durchgeführt. Diese Methode ist im technischen Maßstab jedoch kaum durchführbar. Die erhaltene Schmelze ist stark verunreinigt und fällt nach dem Erkalten als feste, harte Masse an, die zerkleinert und gereinigt werden muß. 4-Phenyl-1,2,4-triazolidin-3,5-dion entsteht nur als Nebenprodukt.

Eine andere Herstellungsmethode für in 4-Stellung substituierte 1,2,4-Triazolidin-3,5-dione ist die Cyclisierung von 1-Alkoxycarbonyl-4-phenyl- (bzw. -4-n-butyl)-semicarbaziden, wie sie im Archiv der Pharmazie 294, 370 (1961) beschrieben wird. Die Ausbeuten an gewünschten Endprodukt liegen bei 80 bis 95%. Eine industrielle Verwertbarkeit dieses Verfahrens ist jedoch bisher am hohen Preis der Ausgangsverbindungen und wegen der Hydrolyseempfindlichkeit der Zwischenprodukte gescheitert.

Aus der FR-PS 1 447 532 ist die Herstellung von 3,5-Dimercapto-1,2,4-triazolen bekannt, die in 4-Stellung unsubstituiert oder durch Alkyl-, Alkenyl-, Aralkyl- oder Arylgruppen substituiert sind. Die Herstellung erfolgt aus den entsprechenden, gegebenenfalls in 1-Stellung substituierten oder 1,6-Stellung disubstituierten Hydrazodithiocarbonsäureamiden (= 1,6 disubstituierte Bis-thioharnstoffe) durch Cyclisierung in wäßriger Lösung in Gegenwart von äquivalenten oder überschüssigen Mengen starker anorganischer oder organischer Basen unter Ammoniak- bzw. Aminabspaltung. Die Ausbeuten an Endprodukten liegen zwischen ca. 40 und 84% der Theorie.

Demgegenüber besteht die Aufgabe vorliegender Erfindung in der Bereitstellung von in 4-Stellung durch gewisse Alkyl, Aryl, Cycloalkyl oder Aralkyl substituierten 1,2,4-Triazolidin-3,5-dionen, und der Kernpunkt der Lösung dieser Aufgabe liegt in der Verwendung spezieller Lösungsmittel bei der Cyclisierungsreaktion. Das ist aus der Entgegenhaltung weder zu entnehmen, noch wird es durch sie nahegelegt.

Überträgt man die Verfahrensweisen in der französischen Patentschrift auf die schwefelfreien analogen Verbindungen, d. h., auf Hydrazodicarbonamid bzw. auf eine Mischung aus ca. 1 Mol Hydrazodicarbonamid und 1 Mol primäres Amin bzw. auf N-substituiertes Hydrazodicarbonamid, so erhält man außerordentlich unbefriedigende Ergebnisse.

Aus Canadian J. Chem. 50 (1972) Seiten 2661–66 ist es bekannt, Hydrazodicarbonamid und Anilin-Hydrochlorid (Molverhältnis 1 : 1) in Kerosin in 7 Stunden bei t = 211° C zu 62% in Phenylurazol zu überführen (vergl. darin Tabelle 2, Versuch 9). Dabei muß das eingesetzte Amin als Hydrochlorid vorliegen, was bekanntlich beim Arbeiten in technischem Maßstab zu Korrosionsproblemen in den Reaktionsapparaturen und bei der Aufarbeitung (Neutralisation) wegen der großen Mengen an anfallenden Salzen zu Abwasserproblemen führt. Demgegenüber zeigt das erfindungsgemäße Verfahren überraschende Vorteile.

Auch durch die in C.A. 67 (1967) Nr. 116858y und C.A. 51 (1955) 12983d offenbarten Verfahren wird der Erfindungsgegenstand nicht nahegelegt.

Gegenstand der Erfindung ist ein Verfahren zur Herstellung von 1,2,4-Triazolidin-3,5-dionen der allgemeinen Formel I

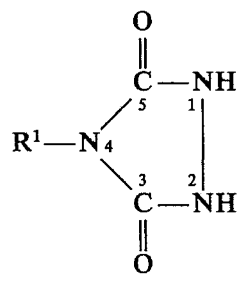

(I)

worin

$R^1$ einen einwertigen linearen oder verzweigten aliphatischen $C_1-C_{30}$, einen einwertigen cycloaliphatischen $C_5-C_{21}$, einen einwertigen aliphatisch-aromatischen $C_7-C_{17}$, oder einen einwertigen aromatischen $C_6-C_{21}$ Rest bedeutet, die vorgenannten Reste können durch mindestens eine der Gruppen Alkoxycarbonyl mit $C_1-C_4$ in der Alkoxygruppe, CN, $NO_2$, Alkylmercaptogruppe mit $C_1-C_4$ in der Alkylgruppe, Dialkylaminogruppe mit $C_1-C_6$ in jeder Alkylgruppe, Halogene und im Falle der aromatischen Reste außer den vorgenannten Substituenten auch durch mindestens eine $C_1-C_4$-Alkylgruppe substituiert sein, die vorgenannten aliphatischen Reste können ferner durch ein oder mehrere Sauerstoffatome oder

tertiäre Stickstoffatome, die vorgenannten mehrkernigen aliphatisch-aromatischen, mehrkernigen cycloaliphatischen und mehrkernigen aromatischen Reste durch mindestens eine Alkylgruppe mit 1—4 C-Atomen, durch mindestens ein Sauerstoffatom oder tertiäres Stickstoffatom unterbrochen sein,

dadurch gekennzeichnet, daß man 0,9 bis 1,1 Mol Hydrazodicarbonamid oder 0,9 bis 1,1 Mol 1,2,4-Triazolidin-3,5-dion mit einem Mol eines primären Amins der Formel II

$$R^1-NH_2 \qquad (II)$$

worin

$R^1$ die oben angegebene Bedeutung besitzt,

in Gegenwart von N-Methylpyrrolidon oder Sulfolan oder deren Mischungen bei Temperaturen von 150 bis 280°C und Drücken von 50 mbar bis 5 bar, gegebenenfalls in Gegenwart eines sauren oder basischen Katalysatoren unter Abspaltung von Ammoniak umsetzt.

Zu den gleichen Verbindungen der Formel I gelangt man auch, wenn anstelle der Ausgangsmaterialien Hydrazodicarbonamid und primäres Amin bzw. 1,2,4-Triazolidin-3,5-dion und primäres Amin ein monosubstituiertes Hydrazodicarbonamid der Formel III

$$H_2N-CO-NH-NH-CO-NH-R^1 \qquad (III)$$

worin

$R^1$ die für Formel I angegebene Bedeutung hat,

unter den gleichen Bedingungen, wie vorstehend angegeben, erhitzt wird.

Als Substituenten an $R^1$ kommen in Betracht: Alkoxycarbonylgruppen mit $C_1-C_4$ in der Alkoxygruppe, CN, $NO_2$, Alkylmercaptogruppen mit $C_1-C_4$ in der Alkylgruppe, Dialkylaminogruppen mit vorzugsweise $C_1-C_6$ in jeder Alkylgruppe, Halogene (vorzugsweise Fluor, Chlor, Brom) und im Falle der aromatischen Reste außer den vorgenannten Substituenten auch niedere Alkylgruppen mit $C_1-C_4$.

Besonders bevorzugt sind Verbindungen der Formel I, in denen der Rest $R^1$ unsubstituiert ist.

Formelmäßig seien beispielhaft folgende bevorzugte Reste $R^1$ angegeben:

1. $CH_3-(CH_2)_m-$ $\quad m = 0-17$

2. 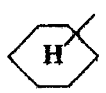

3. 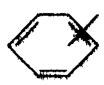

4. 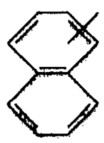

5. 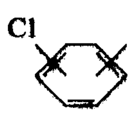

6. $C_4H_9-O-(CH_2)_3-$

7. 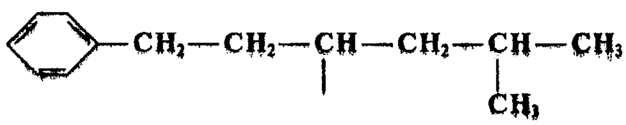

8. 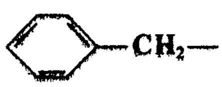

Auf 1 Mol des Amins der Formel II werden 0,9–1,1 Mol Hydrazodicarbonamid bzw. 0,9–1,1 Mol 1,2,4-Triazolidin-3,5-dion eingesetzt.

Das für die Umsetzung mit dem primären Amin der Formel II einzusetzende Hydrazodicarbonamid ist aus der Literatur bekannt und wird bei der Reaktion von 1 Mol Hydrazin mit 2 Mol Harnstoff unter Ammoniakabspaltung in wäßrigem Medium in praktisch quantitativer Ausbeute erhalten. Das dabei als Niederschlag anfallende Hydrazodicarbonamid wird durch Absaugen isoliert und kann als nutschenfeuchte Ware sofort weiterverarbeitet werden, wenn das Restwasser beim Cyclisierungsprozeß abgeführt werden kann. Selbstverständlich kann auch getrocknetes Hydrazodicarbonamid zur weiteren Reaktion verwendet werden. Es ist auch möglich, aus der erhaltenen Suspension aus Hydrazodicarbonamid in Wasser nach Zusatz eines geeigneten Lösungsmittels durch Erhitzen das Wasser abzudestillieren und das verbleibende Hydrazodicarbonamid mit Aminen zu den angegebenen Triazolidin-3,5-dionen umzusetzen.

Die für die Herstellung der Verbindungen der Formel I einzusetzenden N-monosubstituierten Hydrazodicarbonamide der Formel III werden durch Umsetzung von Semicarbazid mit Isocyanaten der Formel IV

$$R^1-NCO \qquad (IV)$$

erhalten, worin

$R^1$ die für Formel I angegebene Bedeutung besitzt.

Im allgemeinen ist es zweckmäßig, die Umsetzung von Semicarbazid und Isocyanat zu den monosubstituierten Hydrazodicarbonamiden der Formel III in einem Lösungs- oder Verdünnungsmittel durchzuführen, wobei dann die Ausgangsmaterialien, im wesentlichen in äquivalenten Mengenverhältnissen, (1 Mol Semicarbazid = 1 OCN-Gruppe) sowohl gelöst als auch nur suspendiert sein können. Selbstverständlich ist es ebenso möglich, die Umsetzung ohne Anwesenheit eines Lösungs- oder Verdünnungsmittels durchzuführen. Für dieses Verfahren geeignete Lösungsmittel sind z. B. aromatische Kohlenwasserstoffe, chlorierte aromatische Kohlenwasserstoffe, Benzonitril, aliphatische Kohlenwasserstoffe, Ester und Ketone. Besonders geeignet sind Toluol, Xylol, Mesitylen, Chlorbenzol, Dichlorbenzol, N-Methylpyrrolidon, Dimethylformamid, Dimethylacetamid, Hexamethylphosphorsäuretriamid, Tetramethylharnstoff, Nitromethan und Nitrobenzol; es ist aber auch möglich, die Reaktion bei niedrigeren Temperaturen in Wasser oder niederen Alkoholen durchzuführen.

Die Reaktion kann bei –30 bis 150° C durchgeführt werden.

Bevorzugt arbeitet man bei –20 bis 100° C, besonders bevorzugt bei –10 bis 80° C.

Es sei dahingestellt, nach welchen Reaktionsmechanismen die Varianten des erfindungsgemäßen Verfahrens im einzelnen ablaufen. Alle Varianten führen in einfacher, glatter und reproduzierbarer Weise und in guten Ausbeuten zu den Verbindungen der Formel I.

Denkbar, ja sogar wahrscheinlich ist, daß bei der Umsetzung von Hydrazodicarbonamid mit primären Amin unter Abspaltung von Ammoniak bzw. bei der Umsetzung von 1,2,4-Triazolidin-3,5-dion mit primären Amin unter Ringöffnung zunächst monosubstituiertes Hydrazodicarbonamid der Formel III gebildet wird, aus dem unter Abspaltung von Ammoniak unter Cyclisierung die Verbindungen der Formel I erhalten werden.

Bei allen Verfahrensvarianten liegt die Reaktionstemperatur zwischen 150 und 280° C, vorzugsweise zwischen 170 und 250° C und insbesondere zwischen 175 und 220° C. Je höher die Temperatur ist, desto schneller verläuft die Reaktion, allerdings nimmt die Gefahr der Bildung unerwünschter Nebenprodukte sowie die Zersetzung des Lösungsmittels zu.

Die Reaktionszeiten liegen im allgemeinen zwischen 1 und 40 Stunden, können jedoch in Ausnahmefällen auch darüber oder darunter liegen.

Zur Beschleunigung der Reaktion kann es zweckmäßig sein, saure oder basische Katalysatoren zuzugeben. Besonders geeignet sind Metallalkoholate (z. B. Natriummethylat, Zinn(II)octoat) und tert. Amine.

Der Reaktionsdruck liegt normalerweise bei 50 mbar bis 5 bar, wobei bei höherem Druck als bei atmosphärischem Druck zwischendurch das entstehende Ammoniak abgelassen werden muß, so daß die Cyclisierung vorzugsweise bei 300 mbar bis 2 bar Druck durchgeführt wird.

Es kann von Vorteil sein, wenn die Konzentration an abgespaltenem Ammoniak im Reaktionsgefäß niedrig gehalten wird. Dies kann auf jede bekannte Weise erfolgen, z. B. durch Ausblasen mit einem inerten Gas wie Luft, Stickstoff, Kohlendioxid oder Wasserdampf. Auch können niedrigsiedende Lösungsmittel, z. B. aliphatische, aromatische, araliphatische Kohlenwasserstoffe, deren technische Mischungen und chlorierte Kohlenwasserstoffe mit vorzugsweise 1–10 Kohlenwasserstoffatomen wie Cyclohexan, Toluol, Xylole, Petrolether oder Chloroform, die flüssig in den Reaktor gepumpt oder getropft werden, zum Austreiben des Ammoniaks verwendet werden. Der Partialdruck des Ammoniaks kann auch durch Absaugen herabgesetzt werden, indem man bei unteratmosphärischem Druck arbeitet.

Die bei der Umsetzung zu verwendenden organischen Lösungsmittel sollen unter Reaktionsbedingungen eine ausreichende thermische Stabilität aufweisen und chemisch inert gegen Hydrazodicarbonamide bzw. Triazolidin-3,5-dione sein, auch soll der Siedepunkt genügend hoch liegen, damit das Lösungsmittel während der Reaktion nicht abdestilliert.

Geeignete Lösungsmittel sind Methylpyrrolidon oder Sulfolan.

Die mit den polaren Lösungsmitteln, wie vorstehend unter A) aufgeführt, nach der Cyclisierung (Umsetzung) erhaltenen Reaktionsmischungen können während des Abkühlungsvorgangs mit gegen Triazolidin-3,5-dione inerten Lösungsmitteln wie aliphatischen, cycloaliphatischen, araliphatischen oder aromatischen Kohlenwasserstoffen, z. B. Cyclohexan, Toluol, Xylol, aliphatischen oder cycloaliphatischen Alkoholen, z. B. Butanol, Cyclohexanol und daraus abgeleiteten Ethern oder Estern, z. B. Glykolmonomethylether, Essigsäurebutylester, Ketone, z. B. Aceton oder Ethylmethylketon und bei Verwendung von mit Wasser mischbaren polaren Lösungsmitteln auch Wasser, abgemischt werden, so daß die auskristallisierenden Triazolidin-3,5-dione in größeren Ausbeuten bzw. höheren Reinheiten anfallen. Die Zusätze betragen bis zu 500 Gew.-%, bezogen auf polares Lösungsmittel.

Das erfindungsgemäße Verfahren ist sowohl für diskontinuierliche als auch für kontinuierliche Arbeitsweise geeignet. Bei der kontinuierlichen Arbeitsweise wird die Cyclisierung nach bekannten Verfahren, z. B. durch die Benutzung von Kaskaden oder der Verwendung von Röhrenreaktoren, die als solche bekannt sind durchgeführt. Das diskontinuierliche Verfahren ist bevorzugt.

Die glatte Reaktion der abgehandelten Verfahren war überraschend, da zu erwarten war, daß bei der Cyclisierung von Hydrazodicarbonamid in Gegenwart von primären Aminen schwer trennbare Gemische von 1,2,4-Triazolidin-3,5-dionen und 1,2,4-Triazolidin-3,5-dionen der Formel I entstehen würden.

Es war ferner überraschend, daß das 1,2,4-Triazolidin-3,5-dion mit primären Aminen zu 4-substituierten Triazolidin-3,5-dionen reagiert, obwohl es thermisch sehr beständig ist.

Die 1,2,4-Triazolidin-3,5-dione der Formel I sind wertvolle Ausgangsstoffe zur Herstellung von temperaturbeständigen Polymeren. Hieraus hergestellte Dihydroxyalkyl-triazolidin-3,5-dione z. B. finden als vernetzende Komponenten, in temperaturbeständigen Elektroisolierlacken Verwendung, während entsprechende Diglycidyl-triazolidin-3,5-dione z. B. als Vernetzer in Pulverlacken, die nach dem elektrostatischen Pulversprühverfahren angewendet werden, eingesetzt werden. Ferner können 1,2,4-Triazolidin-3,5-dione der Formel I in photographischen Zusammensetzungen eingesetzt werden.

Die in den Beispielen angegebenen Prozente beziehen sich auf das Gewicht.

## Beispiel 1

60 g Hydrazodicarbonamid und 56 g n-Hexylamin werden in 100 ml N-Methylpyrrolidon 6 Stunden bei 150°C, 20 Stunden bei 175°C und 20 Stunden bei 180°C gerührt. Anschließend wird das Lösungsmittel im Vakuum abdestilliert und der Rückstand mit 100 ml 10%iger Natronlauge verrieben. Man saugt vom Niederschlag ab und neutralisiert das Filtrat mit 10%iger Salzsäure. Dabei fällt ein Niederschlag aus, der abgesaugt, mit Wasser gewaschen und im Vakuum getrocknet wird. Man erhält 75 g (81% d. Th.) 4-(n-Hexyl)-1,2,4-triazolidin-3,5-dion als farblose Kristalle vom Fp. 144 – 145°C.

$C_8H_{15}N_3O_2$ (185,2)

| | | | |
|---|---|---|---|
| ber. | C 51,87, | H 8,16, | N 22,69%, |
| gef. | C 51,9, | H 8,4, | N 22,6%. |

MS (m/e): Molpeak 185.

## Beispiel 2

10,1 g Triazolidin-3,5-dion und 10 g Cyclohexylamin werden in 20 ml N-Methylpyrrolidon 4 Stunden bei 175°C und 8 Stunden bei 180°C gerührt. Anschließend destilliert man das Lösungsmittel im Vakuum ab, verreibt den Rückstand mit 10 ml 10%iger Natronlauge und saugt vom Niederschlag ab. Das Filtrat wird mit 10%iger HCl neutralisiert. Dabei fällt ein Niederschlag aus, der abgesaugt und mit Wasser gewaschen wird. Man erhält 6,6 g (35% d. Th.) 4-Cyclohexyl-1,2,4-triazolidin-3,5-dion als farblose Kristalle vom Fp. 240 – 245°C.

$C_8H_{13}N_3O_2$ (183,2)

| | | | |
|---|---|---|---|
| ber. | C 52,45, | H 7,15, | N 22,94%, |
| gef. | C 52,5, | H 7,2, | N 22,7%. |

Beispiel 3

53,5 g Benzylamin und 59 g Hydrazodicarbonamid werden in 100 ml N-Methylpyrrolidon 4 Stunden bei 175°C und 5 Stunden bei 200°C gerührt. Anschließend destilliert man das Lösungsmittel im Vakuum ab, verreibt den Rückstand mit 50 ml 10%iger Natronlauge und saugt vom Rückstand ab. Das Filtrat wird mit 10%iger Salzsäure neutralisiert. Dabei entsteht ein Niederschlag, der abgesaugt und mit Wasser gewaschen wird. Man erhält 67 g (70% d. Th.) 4-Benzyl-1,2,4-triazolidin-3,5-dion als farblose Kristalle vom Fp. 185—188° C ($CH_3CN$).

$C_9H_9N_3O_2$ (191,2)

| | | | |
|---|---|---|---|
| ber. | C 56,54, | H 4,74, | N 21,98%, |
| gef. | C 56,8, | H 4,7, | N 22,2%. |

$^1$H-NMR (d-DMSO): =5,60 (s; 2H), 7,33 (s; 5$H_{arom.}$) 10,1 ppm (breit; 2H).
IR (KBr): 1776, 1674 $cm^{-1}$ (C=O).

Beispiel 4

118 g Hydrazodicarbonamid und 100 g Cyclohexylamin werden in 100 ml N-Methylpyrrolidon 2 Stunden bei 160°C, 7 Stunden bei 175°C und 10 Stunden bei 200°C gerührt. Anschließend wird das Lösungsmittel im Vakuum abdestilliert und der Rückstand mit 100 ml 10%iger Natronlauge verrieben. Man saugt vom Niederschlag ab und neutralisiert das Filtrat mit 10%iger Salzsäure. Dabei fällt ein Niederschlag aus, der abgesaugt, mit Wasser gewaschen und im Vakuum getrocknet wird. Man erhält 132 g (76% d. Th.) 4-Cyclohexyl-1,2,4-triazolidin-3,5-dion identisch mit der Verbindung hergestellt nach Beispiel 2.

Beispiel 5

360 g Hydrazodicarbonamid und 807 g Stearylamin und 1 ml Zinn(II)-dioctoat werden in 1 l N-Methylpyrrolidon 4 Stunden bei 175°C und 6 Stunden bei 200°C gerührt. Beim Abkühlen fällt ein Niederschlag aus, der abgesaugt und mit Wasser gewaschen wird. Man erhält 955 g (90% d. Th.) 4-Stearyl-1,2,4-triazolidin-3,5-dion als farblose Kristalle vom Fp. 118—120° C.

$C_{20}H_{39}N_3O_2$ (353,6)

| | | | |
|---|---|---|---|
| ber. | C 67,94, | H 11,12, | N 11,89%, |
| gef. | C 67,9, | H 11,1, | N 11,9%. |

MS (m/e): Molpeak 353.

Beispiel 6

Zu einer Lösung von 111,5 g Semicarbazidhydrochlorid in 700 g Wasser wird so lange in kleinen Portionen Soda zugesetzt, bis keine Gasentwicklung mehr zu beobachten ist.

Dann wird bei 40°C eine Lösung aus 119 g Phenylisocyanat in 100 g Aceton zugetropft. Zur Vervollständigung der Reaktion wird 2 Stunden bei 40° C nachgerührt und der gebildete Niederschlag durch Absaugen isoliert.

Der über Nacht an der Luft getrocknete Niederschlag wird in 300 g Sulfolan suspendiert und auf 205°C aufgeheizt, wobei ab 160°C durch Anlegen eines Wasserstrahlvakuums von 420 mbar das freiwerdende Ammoniak abgezogen wird. Nach einer Reaktionszeit von 5 Stunden wird bei einem Druck von 0,3 mbar das Lösungsmittel größtenteils entfernt und der verbliebene Rückstand aus n-Butanol umkristallisiert. Nach dem Absaugen und Trocknen werden 134 g 4-Phenyl-1,2,4-triazolidin-3,5-dion vom Fp. = 202—203° C (Lit. 203° C) erhalten.

Beispiel 7

131 g 3-Butoxypropylamin und 120 g Hydrazodicarbonamid werden in 300 ml N-Methylpyrrolidon 1 Stunde bei 150°C, 2 Stunden bei 175°C und 6 Stunden bei 200°C gerührt. Beim Abkühlen fällt ein Niederschlag aus, der abgesaugt und aus Cyclohexan umkristallisiert wird. Man erhält 152 g (75% d. Th.) 4-(3-Butoxypropyl)-1,2,4-triazolidin-3,5-dion als farblose Kristalle vom Fp. 83—85° C.

$C_9H_{17}N_3O_3$ (215,2)

| | | | |
|---|---|---|---|
| ber. | C 50,22, | H 7,96, | N 19,52%, |
| gef. | C 50,2, | H 8,1, | N 19,8%. |

$^1$H-NMR($CDCl_3$) = 0.90 (t, 3H; J = 6 Hz), 1.0 – 2.2 (m, 6H), 3.2 – 3.9 (m, 6H), 9,21 ppm (s, 2H).

## Beispiel 8

191 g 3-Amino-5-methyl-1-phenylhexan und 120 g Hydrazocarbonamid werden in 250 ml N-Methylpyrrolidon 1 Stunde bei 150° C, 1 Stunde bei 175° C und 2 Stunden bei 200° C gerührt. Beim Abkühlen fällt ein Niederschlag aus der abgesaugt und aus Cyclohexan umkristallisiert wird. Man erhält 252 g (92% d. Th.) 4-(5-Methyl-1-phenylhexyl-3)-1,2,4-triazolidin-3,5-dion als farblose Kristalle vom Fp. 114 – 115° C.

$C_{15}H_{21}N_3O_3$ (275.3)

| | | | |
|---|---|---|---|
| ber. | C 65,43, | H 7,96, | N 15,25%, |
| gef. | C 65,2, | H 7,4, | N 15,4%. |

IR(KBr): 1767, 1671 $cm^{-1}$ (C = O).

## Beispiel 9

111,5 g Semicarbazidhydrochlorid werden in 250 ml $H_2O$ gelöst und mit Soda neutralisiert. Anschließend werden bei Raumtemperatur unter Kühlung und starkem Rühren 169 g 1-Naphthylisocyanat in 100 ml Dioxan zugetropft. Die Mischung wird 1 Stunde bei Raumtemperatur nachgerührt. Man saugt vom Niederschlag ab, und wäscht ihn mit $H_2O$.

Der feuchte Niederschlag wird in 1 l Sulfolan suspendiert und so lange bei 150° C gerührt, bis kein $H_2O$ mehr abdestilliert. Anschließend wird die Suspension 3 Stunden bei 210° C und 300 mbar gerührt. Beim Abkühlen entsteht ein Niederschlag, der abgesaugt und verworfen wird. Die Mutterlauge wird im Hochvakuum eingeengt und der Rückstand mit 300 ml 10%iger Natronlauge extrahiert. Beim Ansäuern der alkalischen Lösung mit Salzsäure entsteht ein Niederschlag, der abgesaugt und mit Wasser gewaschen wird.

Man erhält 123 g (54% d. Th.) 4-Naphthyl-1,2,4-triazolidin-3,5-dion als farblose Kristalle vom Fp. 283 – 286° C.

$C_{12}H_9N_3O_2$ (227.2)

| | | | |
|---|---|---|---|
| ber. | C 63,43, | H 3,99, | N 18,50%, |
| gef. | C 63,5, | H 3,9, | N 18,7%. |

$^1$H-NMR ($d_7$-DMF) $\delta$ = 7.4 – 8.2 (m, 7H arom.) 10.43 ppm (s, 2H).

## Beispiel 10

Zu einer Lösung von 111,5 g Semicarbazidhydrochlorid in 1000 g Wasser wird in kleinen Portionen so lange Soda zugesetzt, bis keine Gasentwicklung mehr zu beobachten ist. Dann wird bei 35 bis 40° C eine Lösung aus 153,5 g p-Chlorphenylisocyanat in 200 g Dioxan zugetropft. Zur Vervollständigung der Reaktion wird 2 Stunden nachgerührt und der Niederschlag durch Absaugen isoliert.

Der über Nacht an der Luft getrocknete Niederschlag wird in 500 g N-Methylpyrrolidon suspendiert und auf 200° C aufgeheizt, wobei ab 160° C durch Überleiten eines Stickstoffstromes das freiwerdende Ammoniak entfernt wird.

Nach Beendigung der Reaktion wird das Lösungsmittel durch Anlegen eines Wasserstrahlvakuums größtenteils entfernt, der verbliebene Rückstand in Natronlauge gelöst, von unlöslichen Bestandteilen filtriert und die alkalische Lösung mit Salzsäure auf pH 2 gestellt. Nach dem Absaugen und Trocknen werden 146 g 4-(p-Chlorphenyl)-1,2,4-triazolidin-3,5-dion vom Fp = 234 – 236° C (aus Ethanol) erhalten, dessen Struktur mittels IR- und NMR-Spektrum sowie der Elementaranalyse bewiesen wird.

$C_8H_6ClN_3O_2$ (211.6)

| | | | | |
|---|---|---|---|---|
| ber. | C 45,41, | H 2,86, | Cl 16,76, | N 19,86%, |
| gef. | C 45,4, | H 2,8, | Cl 16,9, | N 19,7%. |

Beispiel 11

111,5 g Semicarbazidhydrochlorid werden in 400 ml $H_2O$ gelöst und mit Soda neutralisiert. Anschließend werden unter starkem Rühren bei Raumtemperatur 57 g Methylisocyanat, gelöst in 300 ml Dioxan innerhalb von 1 Stunde zugetropft. Man rührt 2 Stunden bei Raumtemperatur nach und saugt vom entstandenen Niederschlag ab. Der Niederschlag wird in 1 l N-Methylpyrrolidon suspendiert und 10 Stunden bei 200°C und 300 mbar pyrrolysiert. Anschließend wird das Lösungsmittel im Vakuum abdestilliert und der Rückstand aus Ethanol umkristallisiert. Man erhält 96 g (83% d. Th.) 4-Methyl-1,2,4-triazolidin-3,5-dion als farblose Kristalle vom Fp. 230–232°C. (Lit. 232–233°C).

**Patentansprüche**

1. Verfahren zur Herstellung von 1,2,4-Triazolidin-3,5-dionen der allgemeinen Formel I

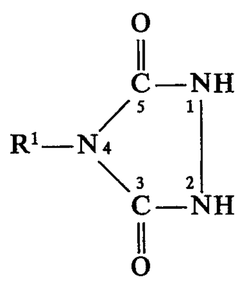

(I)

worin

$R^1$ einen einwertigen linearen oder verzweigten aliphatischen $C_1-C_{30}$, einen einwertigen cycloaliphatischen $C_5-C_{21}$, einen einwertigen aliphatisch-aromatischen $C_7-C_{17}$, oder einen einwertigen aromatischen $C_6-C_{21}$ Rest bedeutet, die vorgenannten Reste können durch mindestens eine der Gruppen Alkoxycarbonyl mit $C_1-C_4$ in der Alkoxygruppe, CN, $NO_2$, Alkylmercaptogruppe mit $C_1-C_4$ in der Alkylgruppe, Dialkylaminogruppe mit $C_1-C_6$ in jeder Alkylgruppe, Halogene und im Falle der aromatischen Reste außer den vorgenannten Substituenten auch durch mindestens eine $C_1-C_4$-Alkylgruppe substituiert sein, die vorgenannten aliphatischen Reste können ferner durch ein oder mehrere Sauerstoffatome oder tertiäre Stickstoffatome, die vorgenannten mehrkernigen aliphatisch-aromatischen, mehrkernigen cycloaliphatischen und mehrkernigen aromatischen Reste durch mindestens eine Alkylgruppe mit 1–4 C-Atomen, durch mindestens ein Sauerstoffatom oder tertiäres Stickstoffatom unterbrochen sein,

dadurch gekennzeichnet, daß man 0,9 bis 1,1 Mol Hydrazodicarbonamid oder 0,9 bis 1,1 Mol 1,2,4-Triazolidin-3,5-dion mit einem Mol eines primären Amins der Formel II

$$R^1-NH_2 \qquad (II)$$

worin

$R^1$ die oben angegebene Bedeutung besitzt,

in Gegenwart von N-Methylpyrrolidon oder Sulfolan oder deren Mischungen bei Temperaturen von 150 bis 280°C und Drücken von 50 mbar bis 5 bar, gegebenenfalls in Gegenwart eines sauren oder basischen Katalysatoren unter Abspaltung von Ammoniak umsetzt.

2. Verfahren nach Anspruch 1, dadurch gekennzeichnet, daß man ein monosubstituiertes Hydrazodicarbonamid der Formel (III)

$$H_2N-CO-NH-NH-CO-NH-R^1 \qquad (III)$$

worin

$R^1$ die in Formel I des Anspruchs 1 angegebene Bedeutung hat,

in Gegenwart von N-Methylpyrrolidon oder Sulfolan oder deren Mischungen bei Temperaturen von 150 bis 280°C und Drücken von 50 mbar bis 5 bar, gegebenenfalls in Gegenwart eines sauren oder basischen Katalysators unter Abspaltung von Ammoniak erhitzt.

**Claims**

1. Process for the preparation of 1,2,4-triazolidine-3,5-diones of the general formula I

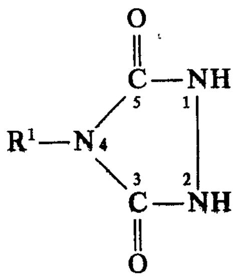

(I)

wherein

$R^1$ denotes a monofunctional linear or branched aliphatic $C_1-C_{30}$ radical, a monofunctional cycloaliphatic $C_5-C_{21}$ radical, a monofunctional aliphatic-aromatic $C_7-C_{17}$ radical or a monofunctional aromatic $C_6-C_{21}$ radical, the aforementioned radicals can be substituted by at least one of the groups alkoxycarbonyl with $C_1-C_4$ in the alkoxy group, CN, $NO_2$, an alkylmercapto group with $C_1-C_4$ in the alkyl group, a dialkylamino group with $C_1-C_6$ in each alkyl group, halogens and, in the case of the aromatic radicals, besides the aforementioned substituents also by at least one $C_1-C_4$-alkyl group, the aforementioned aliphatic radicals can furthermore be interrupted by one or more oxygen atoms or tertiary nitrogen atoms, the aforementioned polynuclear aliphatic-aromatic, polynuclear cycloaliphatic and polynuclear aromatic radicals can be interrupted by at least one alkyl group with 1–4 C atoms, or by at least one oxygen atom or tertiary nitrogen atom,

characterised in that 0.9 to 1.1 mol of hydrazodicarbonamide or 0.9 to 1.1 mol of 1,2,4-triazolidine-3,5-dione is reacted with one mol of a primary amine of the formula II

$$R^1-NH_2 \qquad (II)$$

wherein

$R^1$ has the meaning indicated above,

in the presence of N-methylparrolidone or sulfolane or mixtures thereof, at temperatures of 150 to 280° C and pressures of 50 mbars to 5 bars, optionally in the presence of an acid or basic catalyst, the reaction being accompanied by the elimination of ammonia.

2. Process according to Claim 1, characterised in that a monosubstituted hydrazodicarbonamide of the formula (III)

$$H_2N-CO-NH-NH-CO-NH-R^1 \qquad (III)$$

wherein

$R^1$ has the meaning indicated in formula I of Claim 1,

is heated, in the presence of N-methylpyrrolidone or sulfolane or mixtures thereof, at temperatures of 150 to 280° C and pressures of 50 mbars to 5 bars, optionally in the presence of an acid or basic catalyst, the reaction being accompanied by the elimination of ammonia.

**Revendications**

1. Procédé de préparation de 1,2,4-triazolidine-3,5-diones de formule générale I

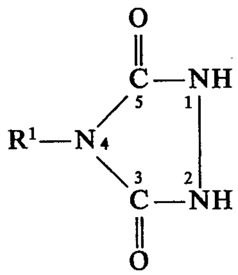

(I)

dans laquelle

$R^1$ représente un reste aliphatique linéaire ou ramifié monovalent en $C_1-C_{30}$, un reste cycloaliphatique monovalent en $C_5-C_{21}$, un reste aliphatique-aromatique monovalent en $C_7-C_{17}$ ou un reste aromatique monovalent en $C_6-C_{21}$, ces restes pouvant être substitués par au moins un des groupes alcoxycarbonyle en $C_1-C_4$ dans la partie alcoxy, CN, $NO_2$, alkylmercapto en $C_1-C_4$ dans la partie alkyle, dialkylamino en $C_1-C_6$ dans chaque groupe alkyle, des halogènes et en outre, dans le cas des restes aromatiques, en plus des substituants mentionnés ci-dessus, par au moins un groupe alkyle en $C_1-C_4$, les restes aliphatiques mentionnés ci-dessus pouvant en outre être interrompus par un ou plusieurs atomes d'oxygène ou atomes d'azote tertiaire, les restes aliphatiques-aromatiques polycycliques, cycloaliphatiques polycycliques et aromatiques polycycliques mentionnés ci-dessus par au moins un groupe alkyle en $C_1-C_4$, par au moins un atome d'oxygène ou atome d'azote tertiaire,

caractérisé en ce que l'on fait réagir 0,9 à 1,1 mole d'hydrazodicarboxyamide ou 0,9 à 1,1 mole de 1,2,4-triazolidine-3,5-dione avec 1 mole d'une amine primaire de formule II

$$R^1-NH_2 \qquad \text{(II)}$$

dans laquelle

$R^1$ a la signification indiquée ci-dessus,

en présence de N-méthylpyrrolidone ou de sulfolanne ou leurs mélanges, à des températures de 150 à 280°C et des pression de 50 mbar à 5 bar, éventuellement en présence d'un catalyseur acide ou basique, avec séparation d'ammoniac.

2. Procédé selon la revendication 1, caractérisé en ce que l'on chauffe un hydrazodicarboxamide monosubstitué de formule III

$$H_2N-CO-NH-NH-CO-NH-R^1 \qquad \text{(III)}$$

dans laquelle

$R^1$ a la signification indiquée en référence à la formule I de la revendication 1,

en présence de N-méthylpyrrolidone ou de sulfolanne ou de leurs mélanges à des températures de 150 à 280°C et des pressions de 50 mbar à 5 bar, éventuellement en présence d'un catalyseur acide ou basique, avec séparation d'ammoniac.